(19) Europäisches Patentamt
European Patent Office
Office européen des brevets

(11) Veröffentlichungsnummer: **0 308 791 B1**

(12) # EUROPÄISCHE PATENTSCHRIFT

(45) Veröffentlichungstag der Patentschrift: **14.11.90**

(51) Int. Cl.⁵: **C07D 301/12**, C07D 303/02

(21) Anmeldenummer: **88114989.2**

(22) Anmeldetag: **14.09.88**

(54) **Verfahren zur katalytischen Epoxidation von Olefinen mit Wasserstoffperoxid.**

(30) Priorität: **21.09.87 DE 3731690**

(43) Veröffentlichungstag der Anmeldung:
**29.03.89 Patentblatt 89/13**

(45) Bekanntmachung des Hinweises auf die Patenterteilung:
**14.11.90 Patentblatt 90/46**

(84) Benannte Vertragsstaaten:
**AT BE CH DE ES FR GB IT LI NL**

(56) Entgegenhaltungen:
**EP-A- 0 074 259**
**DE-A- 3 539 268**
**DE-A- 3 602 254**
**DE-B- 2 803 791**

(73) Patentinhaber: **Degussa Aktiengesellschaft, Weissfrauenstrasse 9, D-6000 Frankfurt am Main 1(DE)**

(72) Erfinder: **Buchler, Johann, Prof. Dr., Richard-Wagner-Weg 93, D-6100 Darmstadt(DE)**
Erfinder: **Schmidt, Manfred, Dr., Unter-Haitzergasse 19, D-6460 Gelnhausen(DE)**
Erfinder: **Prescher, Günter, Dr., 10 Sherwood Drive, Larchmont, N.Y. 10538(US)**

EP 0 308 791 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die Erfindung betrifft ein Verfahren zur katalytischen Epoxidation von Olefinen mit Wasserstoffperoxid in Anwesenheit eines Übergangsmetallporphyrinkomplexes, bei dem ein erforderlicher Ladungsausgleich durch ein Anion erfolgt.

Olefinoxide (Oxirane) sind Verbindungen von beträchtlicher industrieller Bedeutung. Sie finden Verwendung auf dem Gebiet der Lacke, zur Herstellung von Polyethern, Polyurethanen, Epoxidharzen, Detergentien, Glykolen und einer Vielzahl von organischen Zwischenprodukten (siehe US-PS 2 412 136 sowie DE-AS 11 39 477).

Zur Epoxidation von Olefinen sind schon verschiedene Verfahren bekannt. So lassen sich Oxirane nach der Chlorhydrinmethode durch Umsetzung von Olefinen mit Chlor oder Natriumhypochlorit in alkalischem Medium und nachfolgender Behandlung mit Basen herstellen. Ein grundsätzlicher Nachteil dieses Verfahrens besteht in der Bildung salzhaltiger umweltbelastender Abwässer und unerwünschter chlorierter Nebenprodukte (siehe Ullmann's Enzyklopädie der technischen Chemie, Band 10, Seite 565 (3. Auflage)).

Ein weiterer Prozeß beruht auf der Umsetzung von Olefinen mit organischen Hydroperoxiden in Gegenwart eines Katalysators (siehe DE-AS 14 68 012). Dieses zweite Syntheseprinzip hat den entscheidenden Nachteil, daß aufgrund der Stöchiometrie der Epoxidationsreaktion das üblicherweise teure organische Hydroperoxid (ROOH, wobei R z. B. einen niedermolekularen Rest, wie t-Butyl oder Cumyl, bedeuten kann) während der Reaktion gemäß

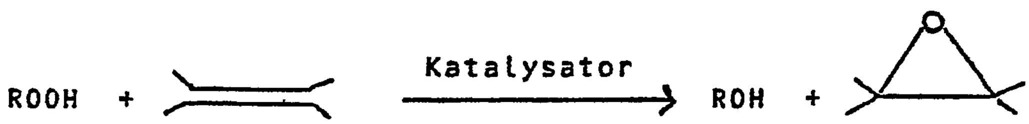


in große Mengen des entsprechenden Alkohols (ROH) umgewandelt wird. Falls der entsprechende Alkohol nicht verwertet werden kann, muß er vom Verfahrensprodukt abgetrennt und entsorgt oder über mehrere Verfahrensstufen in das entsprechende Hydroperoxid zurückverwandelt werden, wodurch das Epoxidationsverfahren auch in wirtschaftlicher Hinsicht aufwendig wird.

Ein weiteres Verfahren beruht auf der Verwendung von organischen Persäuren, die man durch Luftoxidation der entsprechenden Aldehyde oder aus Carbonsäuren mit Wasserstoffperoxid erhält (siehe BE-PS 535 068). Der Einsatz dieser organischen Percarbonsäuren ist wegen deren Zersetzlichkeit stets mit einem Risiko behaftet und erfordert deshalb aufwendige Vorsichtsmaßnahmen in bezug auf Verfahrensführung und Apparateaufbau. Zusätzlich entstehen bei Epoxidierungen mit organischen Persäuren immer große Mengen der entsprechenden Carbonsäuren, die in stöchiometrischer Menge oder überstöchiometrischer Menge abgetrennt und entsorgt oder zurückgeführt werden müssen.

Die geschilderten Nachteile lassen sich durch Verwendung von Wasserstoffperoxid als Epoxidationsmittel beheben, da hierbei nach der Theorie neben dem Epoxidationsprodukt nur Wasser anfallen sollte. Da die Reaktivität des Wasserstoffperoxids gegenüber Olefinen schwach ist, werden Epoxidationen mit diesem Reagens unter Verwendung von Katalysatoren durchgeführt. Nur für wenige Olefine sind Katalysatoren wie Molybdän- und Wolframverbindungen geeignet. In diesem Zusammenhang wird beispielsweise hingewiesen auf GB-PS 837 464, bei welchem die in J.A.C.S., Band 59, Seiten 2342 bis 2344 (1937) beschriebenen verschiedenen Metallkatalysatoren verwendet werden, auf US-PS 2 786 854, wonach Wolframsäure eingesetzt wird, auf US-PS 2 833 787, wonach saure Salze von Metallen aus der Gruppe VI des Periodensystems der Elemente, z. B. von Wolfram oder Molybdän, angewandt werden, auf BE-PS 860 776, wonach Wolfram- und Molybdän-haltige Verbindungen verwendet werden, auf US-PS 3 993 673, wonach Arsen-haltige Katalysatoren verwendet werden, auf US-PS 953 362, wonach ein Molybdän-haltiger Katalysator angewandt wird, auf US-PS 4 026 908, wonach Quecksilberderivate plus eine Molybdän-, Wolfram-, Vanadin- oder Titanverbindung angewandt wird, auf US-PS 3 806 467, wonach organische und anorganische Zinnverbindungen plus organische oder anorganische Verbindungen, die Molybdän, Wolfram, Vanadin, Selen oder Bor enthalten, eingesetzt werden, auf Bull. Chem. Soc. Jap. 42, Seiten 1604 (1969), wonach Selendioxid angewandt wird und auf US-PS 3 778 451, wonach Molybdän-, Wolfram-, Vanadin-, Niob-, Tantal-, Uran- und Rheniumverbindungen eingesetzt werden.

Diese Stoffe sind zwar katalytisch aktiv, doch haben aus verschiedenen Gründen die damit grundsätzlich ausführbaren Verfahren keinen Eingang in die Technik gefunden. In Verbindung mit Wasserstoffperoxidlösungen wird durch sie entweder das Wasserstoffperoxid rasch zersetzt oder nur eine unbefriedigende Epoxidationsgeschwindigkeit erreicht. Verfahren mit diesen Katalysatoren sind auch insoweit problematisch, als neben dem gewünschten Epoxidationsprodukt häufig größere Mengen an Nebenprodukten, wie Diole und Ketone gebildet werden, deren Abtrennung erhebliche Schwierigkeiten bereiten kann.

Es sind auch schon Versuche unternommen worden, Verfahren zur katalytischen Epoxidation von Olefinen mit anderen Epoxidationsmitteln unter Verwendung von Metallporphyrinkomplexen als Katalysatoren durchzuführen. Als Epoxidationsmittel wurden hierbei Verbindungen wie Jodosobenzol (PhJO) (Groves, J.T.; Nemo, T.E.; Myers, R.S., J. Am. Chem. Soc., 101, 1032 (1979), Alkalimetallhypochlorit, wie NaOCL oder LiOCL (Guilmet, E.; Meunier, B.; Tetrahedron Lett. 1980, 4449) sowie organische Hydroperoxide, wie t-Butylhydroperoxid oder Cumolhydroperoxid (Ledon, H.J.; Durbut, P.; Varescon, F., J. Am. Chem. Soc. 103, 3601 (1981) eingesetzt. Als zur Umsetzung mit diesen Epoxidationsmitteln geeignete Metallkatalysatoren sind z. B. das Chloro-Eisen(III)-tetraphenylporphyrin (ClFe(TPP)), das Chloro-Mangan (III)-tetraphenylporphyrin (ClMn(TPP)) oder das Chloro-Chrom(III)-tetraphenylporphyrin (ClCr(TPP)) vorgeschlagen worden. Mangan(III)-tetraphenylporphyrin wurde auch bereits mit Wasserstoffperoxid als Oxidationsmittel eingesetzt (Renaud, J.-P.; Battioni, P.; Bartoli,J.F.; Mansuy, D., J. Chem. Soc., Chem. Commun., 1985, 888). Allerdings wirken diese Katalysatoren stark zersetzend auf $H_2O_2$, so daß die bezüglich Wasserstoffperoxid erreichbaren Selektivitäten nur sehr gering sind, es sei denn, es werden aufwendig substituierte Porphyrinliganden verwendet. Auch Oxo-Metallporphyrinkomplexe, wie Oxo-chloro(5,10,15,20-tetraphenylporphyrin)-molybdän(V) (O=Mo(TPP)Cl) sind in Verbindung mit organischen Hydroperoxiden schon vorgeschlagen worden. Ein Versuch, anstelle eines organischen Hydroperoxids Wasserstoffperoxid mit einem Katalysator der Zusammensetzung Oxo(5,10,15,20-tetraphenylporphyrin)-molybdän(V)-methoxid zur Epoxidierung des Olefins Cyclohexen zu verwenden, schlug jedoch fehl: Es konnte keine Epoxidation beobachtet werden (F.Varescon, These, L'Université Claude Bernard-Lyon I, 1982).

Es wurde nun gefunden, daß die katalytische Epoxidation von Olefinen mit Wasserstoffperoxid mit sehr hoher Selektivität gelingt, wenn das Olefin in Gegenwart von

- Rhenium-oxo-Komplexen oder
- einer zweikernigen Verbindung des Typs µ-Oxobis [porphyrinatooxorhenium(V)]

mit jeweils

- Octaethylporphyrin oder
- 5,10,15,20-Tetraphenylporphyrin oder
- 5,10,15,20-Tetra(4-pyridyl)-porphyrin

als Liganden, bei denen gegebenenfalls jeweils Wasserstoffatome oder freie Elektronenpaare an den Phenyl- bzw. Pyridylgruppen ein- oder mehrmals substituiert sind durch Halogen, Hydroxy, Carboxy, Cyano, Rhodano, Nitro, $C_1$-$C_6$-Alkyl, Trihalogenmethyl, $C_1$-$C_6$-Alkoxy, $C_1$-$C_6$-Alkansulfonyloxy, Aminocarbonyl, einen oder zwei $C_1$-$C_6$-Alkylreste enthaltendes Aminocarbonyl, $C_1$-$C_6$-Alkylcarbonyl, Amino, Di-$C_1$-$C_6$-Alkylamino, $C_1$-$C_6$-Alkanoylamino, $C_1$-$C_6$-Alkyl-$C_1$-$C_6$-alkanoylamino, $C_1$-$C_6$-Alkansulfonylamino, $C_1$-$C_6$-Alkyl-$C_1$-$C_6$-alkansulfonylamino, Aminosulfonyl, einen oder zwei $C_1$-$C_6$-Alkylreste enthaltendes Aminosulfonyl, $C_1$-$C_6$-Alkoxysulfonyl (-$SO_2$-O-$C_1$-$C_6$-Alkyl), Sulfo oder $C_1$-$C_6$-Alkansulfonyl und zwei dieser Reste auch die Methylendioxygruppe sein können, wobei der Komplex bei den Rhenium-oxo-Komplexen gegebenenfalls am Zentralatom ein Anion aus der Reihe $F^-$, $Cl^-$, $Br^-$, $I^-$, $CH_3O^-$, $C_2H_5O^-$, $C_3H_7O^-$, t-$C_4H_9O^-$, $C_6H_5O^-$, $HO^-$, $AcO^-$, $SCN^-$, $OCN^-$, $ClO_4^-$ trägt, in homogener Phase oder in einem Zweiphasensystem mit Wasserstoffperoxid umgesetzt wird.

Durch die sterischen und elektronischen Effekte der genannten Substituenten am Phenyl- bzw. Pyridylrest des 5,10,15,20-Tetraphenylporphyrins bzw. 5,10,15,20-Tetra(4-pyridyl)porphyrins kann man, angepaßt an das jeweilige Olefin, die katalytischen Eigenschaften steuern und optimieren.

Die erfindungsgemäß zur Durchführung des Verfahrens vorgesehenen Katalysatoren sind zum Teil neue Stoffe. Davon ist eine Reihe nach den bekannten Literaturmethoden in großer Reinheit zugänglich: J.W. Buchler et al., Chem. Ber., 1973, 106, 2710; Liebigs Ann. Chem., 1971, 745, 135; Inorg. Nucl. Chem. Lett., 1972, 8, 1073; K. Rohbock, Dissertation, RTWH Aachen, 1972; H. Stoppa, Dissertation, RTWH Aachen, 1976.

Die verschiedenen Porphyrinliganden werden, sofern sie nicht käuflich sind, nach Adler et al., J. Org. Chem. 32, 476 (1967) bzw. Adler et al., J. Heterocycl. chem. 5, 669 (1968) dargestellt und sofern erforderlich, von Chlorin (Porphyrin mit einem teilhydrierten Pyrrolglied) befreit (K.M. Smith et al., Tetrahedron Lett., 30, 2887 (1973)).

Die neuen, in der gleichzeitig eingereichten Parallelanmeldung P 37 31 689.3-44 beschriebenen Stoffe unter den Rhenium-oxo-Komplexen können nur zum Teil nach der bekannten $Re_2O_7$-Methode hergestellt werden.

In der genannten Parallelanmeldung sind neue Herstellungsverfahren für diese neuen Stoffe bzw. ihre Vorstufe erstmals offenbart, welche sich auch zur Gewinnung der bereits bekannten, für das erfindungsgemäße Verfahren beanspruchten Komplexe eignen.

Die Insertion des Rheniums in das gegebenenfalls substituierte Porphyrinligandensystem gelingt mit überschüssigem $ReCl_5$ in siedendem Trichlorbenzol in sehr guter Ausbeute (85 - 90 %) nach Gleichung I und II

$$\text{(I)} \quad ReCl_5 + H_2(P) \longrightarrow [ReCl_2(P)]^+Cl^- + 2HCl$$

$$\text{(II)} \quad [ReCl_2(P)]^+Cl^- + H_2O \longrightarrow ReO(P)Cl + 2HCl$$

Die Einführung definierter Axialliganden sowie die Bildung des über Sauerstoff verbrückten Zweikernkomplexes erfolgt nach Gleichungen III und IV.

$$\text{(III)} \quad ReO(P)Cl + HX \rightleftharpoons ReO(P)X + HCl$$

$$\text{(IV)} \quad ReO(P)X + H_2O \rightleftharpoons [ReO(P)]_2O + HX$$

Bedeutungen:

P = Porphyrinligand, gegebenenfalls substituiert;
X = beliebiges, einfach negativ geladenes Anion;

Nach dem erfindungsgemäßen Epoxidationsverfahren können Olefine entsprechend der allgemeinen Formel

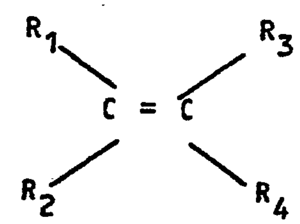

umgesetzt werden, wobei $R_1$ bis $R_4$ identisch oder verschieden sein können und sowohl Wasserstoff oder einen linearen oder verzweigten Alkylrest mit 1 bis 30 Kohlenstoffatomen oder einen Cycloalkylrest mit 3 bis 12 C-Atomen, die als Heteroatome z. B. ein- oder mehrere O-, N- oder S-Atome enthalten können, bedeuten. $R_1$ und $R_2$ oder $R_3$ und $R_4$ können auch durch funktionelle Gruppen substituiert sein, welche im Reaktionsmilieu stabil sind, wie z. B. durch Hydroxy-, Chloro-, Fluoro-, Bromo-, Jodo-, Nitro-, Methoxy-, Alkoxy-, Amino-, Carbonyl-, Ester-, Amido-, Nitrilo-Gruppen. Sie können auch ungesättigt sein, d. h. daß Polyolefine, wie z. B. Diene, Triene und andere Verbindungen mit Doppelbindungen ebenfalls in der vorliegenden Erfindung verwendet werden können, seien sie konjugiert oder nicht.

Unter dieser Voraussetzung kommen unter den Olefinen, welche nach dem vorliegenden Verfahren epoxidiert werden können, beispielsweise die folgenden in Betracht:

Ethylen, Propylen, die Butene, Butadien, die Pentene, Isopren, Hexen(1), Hexen(3), Hepten(1), Okten(1), Diisobutylen, Nonen(1), Tetradecen(1), Pentamyrcen, Camphen, Undecen(1), Dodecen(1), Tridecen(1), Tetradecen(1), Pentadecen(1), Hexadecen(1), Heptadecen(1), Oktadecen(1), Nonadecen(1), Eikosen(1), die Trimeren und Tetrameren des Propylens, die Polybutadiene, die Polyisoprene, Styrol, α-Methylstyrol, Divinylbenzol, Inden, Stilben, Cyclopenten, Cyclohexen, Cyclohepten, Cycloocten, Cyclooctadien, Cyclododecen, Cyclododekatrien, Dicyclopentadien, Methylencyclopropan, Methylencyclopentan, Methylencyclohexan, Vinylcyclohexen, Methallylketon, Allylchlorid, Allylbromid, Acrylsäure, Methacrylsäure, Crotonsäure, Vinylessigsäure, Crotylchlorid, Methallylchlorid, die Dichlorbutene, Allylalkohol, Allylkarbonat, Allylacetat, die Alkyle der Acrylate und Methacrylate, Diallylmaleat, Diallylphthalat, die ungesättigten Öle wie Sojaöl, die ungesättigten Fettsäuren wie Ölsäure, Linolensäure, Balidinsäure, Erucasäure, Oleostearinsäure, Myristinsäure, Palmitinölsäure, Ricinolsäure und deren Ester.

Ein Vorteil der Erfindung besteht darin, daß sich hierbei das als Reaktand benötigte Wasserstoffperoxid in allen handelsüblichen Formen verwenden läßt, nämlich in Form wäßriger Wasserstoffperoxidlösungen mit einem Wasserstoffperoxidgehalt von 30 bis 90 Gew.% oder als reines Wasserstoffperoxid, stärker verdünntes Wasserstoffperoxid, in organischen Lösungsmitteln gelöstes wasserfreies Wasserstoffperoxid oder in Form von Verbindungen, die unter den Reaktionsbedingungen Wasserstoffperoxid freisetzen (Metallperoxide, wie Magnesium- oder Zinkperoxid) sowie Wasserstoffperoxid-Anlagerungsverbindungen (Peroxohydrate), z. B. von Natriumcarbonat, Natriumpyrophosphat und Harnstoff).

Besonders vorteilhaft ist, wenn man als Reaktionsmedium ein organisches Lösungsmittel oder ein Lösungsmittelgemisch verwendet, welches einen Übertritt von als wäßrige Lösung eingesetztem Wasserstoffperoxid in die organische Phase gestattet.

Als organische Lösungsmittel können dafür z. B. Alkyl- oder Cycloalkylester von gesättigten aliphatischen Carbonsäuren mit einer Kohlenstoffzahl von 4 - 8 (siehe z. B. DE-PS 32 25 307, Seite 5), Methylenchlorid, Dioxan, tert.-Butanol, Tetrahydrofuran, Benzol, Äthanol, Chloroform und Methanol verwendet werden.

Als Lösungsmittelgemische kommen z. B. Kombinationen aus einem oder mehreren der obengenannten Carbonsäureester mit Wasser, Methylenchlorid, Dioxan, tert.-Butanol, Tetrahydrofuran, Benzol, Äthanol, Chloroform und/oder Methanol in Frage.

Für die Lösung von wasserfreiem Wasserstoffperoxid hat sich ein Einsatz von Alkyl- oder Cycloalkylestern von gesättigten aliphatischen Carbonsäuren mit einer Kohlenstoffzahl von 4 - 8 besonders bewährt.

Die anzuwendenden Mengen an Katalysator, die im erfindungsgemäßen Verfahren eingesetzt werden, können in einem weiten Bereich liegen. Die im Einzelfall anzuwendende Katalysatorkonzentration kann, entsprechend dem Typ der gewählten, gemäß Erfindung vorgesehenen Rhenium-porphyrinverbindung sowie entsprechend der Reaktivität des jeweils umzusetzenden Olefins gewählt werden.

Sie liegt in einem Konzentrationsbereich, der im allgemeinen 1/10000 bis 1/2 mol, vorzugsweise 1/5000 bis 1/5 mol pro mol Wasserstoffperoxid beträgt.

Ein weiterer Gegenstand der Erfindung ist die mehrfache Verwendung des gebrauchten Katalysators, der nach geeigneter Abtrennung des Reaktionsgemisches für weitere Ansätze eingesetzt werden kann.

Die Reaktionstemperaturen können in einem breiten Bereich liegen. Sie hängen ab von der jeweiligen Aktivität des verwendeten Katalysators, der Reaktivität des verwendeten Olefins, der Neigung des gewünschten Oxirans zur Ringöffnung und der Art des Lösungsmittels. Sie liegen im allgemeinen bei 0 bis 150, vorzugsweise 20 bis 120, insbesondere 20 bis 80° C. Die Reaktionszeiten liegen normalerweise bei 10 Minuten bis 24 Stunden. Die Reaktionen können unter Atmosphärendruck oder bei höheren Drucken durchgeführt werden, solange das Reaktionssystem in flüssiger Phase gehalten werden kann.

Vorzugsweise wird in einem Druckbereich zwischen 1 und 50 bar gearbeitet.

Die mit der Erfindung erzielbaren Vorteile sind:

- Sehr kurze Reaktionszeiten
- Hohe Selektivität (kaum Nebenprodukt)
- Niedrige Katalysatorkonzentration
- Hohe chemische Stabilität des Katalysators, insbesondere gegenüber dem Epoxidationsmittel
- Keine oder nur minimale $H_2O_2$-Zersetzung
- Aus dem Epoxidationsmittel entsteht nur Wasser
- Leichte Abtrennbarkeit und Wiederverwendbarkeit des Katalysators
- Einfacher Verfahrensweg

Die Erfindung wird im folgenden anhand von Ausführungsbeispielen näher erläutert.

Vorbemerkung zu den einzelnen Beispielen:

Die in den Ausführungsbeispielen verwendeten, zum Teil nach der Parallelanmeldung P 37 31 689.3-44 oder nach der Literatur erhältlichen Katalysatoren werden zur Epoxidation unterschiedlicher olefinischer Ausgangsstoffe mit Wasserstoffperoxid gemäß der Erfindung wie folgt eingesetzt:

Man stellt eine Lösung aus Olefin, Katalysator und Lösungsmittel her, erwärmt sie auf eine Temperatur im Bereich von 20 - 100° C und versetzt mit Wasserstoffperoxid (30 bis 90 Gew.%) - Version I - oder man stellt eine Lösung aus Olefin, Wasserstoffperoxid (30 bis 90 Gew.%) und Lösungsmittel her und versetzt diese dann mit Katalysator; dann wird unter Rühren und Erwärmen umgesetzt - Version II.

In einer abgewandelten Ausführungsform, die sich auf die Versionen I und II gleichermaßen anwenden läßt, wird den vorgelegten Komponenten vor Zugabe der restlichen Komponente noch eine kleine Menge eines heterocyclischen Amins, z. B. aus der Familie des Pyridins oder Imidazols zugesetzt. Bei den eingesetzten Olefinen kann unter atmosphärischem Druck gearbeitet werden. Im Verlaufe der Umsetzung werden Proben entnommen und auf ihren Gehalt an Epoxid bzw. $H_2O_2$ analysiert. Die Menge an gebildeten Epoxiden wird entweder durch Gaschromatographie oder Titration, diejenige an Wasserstoffperoxid durch übliche Titration mit Cer(IV)-sulfat ermittelt. Die bei den Versuchen erhaltenen Ergebnisse gehen aus der folgenden Tabelle hervor, wobei die Selektivität wie folgt definiert ist:

$$\text{Selektivität (\%)} = \frac{\text{Mol gebildetes Epoxid (Diol)}}{\text{Mol umgesetztes } H_2O_2} \times 100$$

| Beispiel Nr. | Olefin (mmol) | $H_2O_2$ (mmol) | Katalysator | (mmol) | Reaktionsbeding. Solvent | Temp. (°C) | Zeit (h) | Umsatz $H_2O_2$ (%) | Selektiv. % |
|---|---|---|---|---|---|---|---|---|---|
| 1 | 1,5-COD[1] (36) | 85% in $H_2O$ (12) | ORe(TTP)[3] $OCH_3$ | $12 \times 10^{-2}$ | nPAC[2] | 60 | 6 | 88,7 | 100 |
| 2 | 1,5-COD (36) | 85% in $H_2O$ (12) | ORe(TTP) $OCH_3$ * | $12 \times 10^{-2}$ | nPAC | 60 | 2 | 90,3 | 100 |
| 3** | 1,5-COD (36) | - | ORe(TTP) $OCH_3$ | $12 \times 10^{-2}$ | nPAC | 60 | 6 | 0 | 0 |
| 4 | 1,5-COD (18) | 85 % in $H_2O$ (6) | ORe(TTP) $OClO_3$ | $6 \times 10^{-2}$ | nPAC | 60 | 3 | 90,6 | 92,5 |
| 5 | 2-Methyl-2-buten (36) | 85% in $H_2O$ (12) | ORe(TTP) $OCH_3$ | $12 \times 10^{-2}$ | nPAC | 60 | 6 | 68,1 | 84,3 |
| 6 | 1,5-COD (9) | 85% in $H_2O$ (3) | ORe(TTP)OAc | $3 \times 10^{-2}$ | nPAC | 60 | 2 | 63,9 | 71,6 |
| 7 | Cyclohexen (36) | 85 % in $H_2O$ (12) | ORe(TTP) $OCH_3$ | $12 \times 10^{-2}$ | tert.-Butanol | 60 | 6 | 98,5 | 68,0 |

1) 1,5-Cyclooctadien
2) Essigsäurepropylester
3) TTP = 5,10,15,20-Tetra(p-tolyl)-porphyrin
4) TTP = 5,10,15,20-Tetraphenyl-porphyrin

* Wiederverwendung des Katalysators nach Beispiel 1
** Kontrollbeispiel

| Beispiel Nr. | Olefin (mmol) | $H_2O_2$ (mmol) | Katalysator | (mmol) | Reaktionsbeding. Solvent | Temp. (°C) | Zeit (h) | Umsatz $H_2O_2$ (%) | Selektiv. % |
|---|---|---|---|---|---|---|---|---|---|
| 8 | Cyclohexen (30) | 85% in $H_2O$ (10) | ORe(TTP)[1] Br | 0,10 | tert.But. | 60 | 6 | 70,0 | 82,9 |
| 9 | 1,5-COD[2] (18) | 85% in $H_2O$ (6) | [ReO(TPP)[3]]$_2$O[4] | 0,06 | n-PAC[5] | 60 | 6 | 84,3 | 56,5 |
| 10 | Cyclohexen (36) | 85% in $H_2O$ (12) | ORe(TAP)[6] $OCH_3$ | 0,12 | tert.But. | 60 | 30 | 93,6 | 34,6 |
| 11 | Cyclohexen (36) | 85% in $H_2O$ (12) | ORe(TpCP)[7] $OCH_3$ | 0,12 | tert.But | 60 | 6 | 67,2 | 73,9 |
| 12 | Cyclohexen (36) | 85% in $H_2O$ (12) | ORe(TAP)$OCH_3$ *) | 0,12 | tert.But. | 60 | 6 | 53,2 | 52,8 |

2) 1,5-Cyclooctadien
5) Essigsäure n-propylester
1) TTP = 5,10,15,20-Tetra(p-tolyl)-porphyrin

3) TPP = 5,10,15,20-Tetraphenylporphyrin
4) μ-Oxobis [porphyrinatooxorhenium(V)]
6) TAP = 5,10,15,20-Tetraanisylporphyrin
7) TpCP = 5,10,15,20-Tetra(p-Chlorphenyl-porphyrin

*) Wiederverwendung des Katalysators nach Beispiel 10

## Patentansprüche

1. Verfahren zur katalytischen Epoxidation von Olefinen mit Wasserstoffperoxid,
**dadurch gekennzeichnet,**
daß man das Olefin in Gegenwart von

- Rhenium-oxo-Komplexen oder
- einer zweikernigen Verbindung des Typs µ-Oxobis [porphyrinatooxorhenium(V)]

mit jeweils

- Octaethylporphyrin
- 5,10,15,20-Tetraphenylporphyrin oder
- 5,10,15,20-Tetra(4-pyridyl)-porphyrin

als Liganden, bei denen gegebenenfalls jeweils Wasserstoffatome oder freie Elektronenpaare an den Phenyl- bzw. Pyridylgruppen ein- oder mehrmals substituiert sind durch Halogen, Hydroxy, Carboxy, Cyano, Rhodano, Nitro, $C_1$-$C_6$-Alkyl, Trihalogenmethyl, $C_1$-$C_6$-Alkoxy, $C_1$-$C_6$-Alkansulfonyloxy, Aminocarbonyl, einen oder zwei $C_1$-$C_6$-Alkylreste enthaltendes Aminocarbonyl, $C_1$-$C_6$-Alkylcarbonyl, Amino, Di-$C_1$-$C_6$-Alkylamino, $C_1$-$C_6$-Alkanoylamino, $C_1$-$C_6$-Alkyl-$C_1$-$C_6$-alkanoylamino, $C_1$-$C_6$-Alkansulfonylamino, $C_1$-$C_6$-Alkyl-$C_1$-$C_6$-alkansulfonylamino, Aminosulfonyl, einen oder zwei $C_1$-$C_6$-Alkylreste enthaltendes Aminosulfonyl, $C_1$-$C_6$-Alkoxysulfonyl(-$SO_2$-O-$C_1$-$C_6$-Alkyl), Sulfo oder $C_1$-$C_6$-Alkansulfonyl und zwei dieser Reste auch die Methylendioxygruppe sein können, wobei der Komplex bei den Rhenium-oxo-Komplexen gegebenenfalls zm Zentralatom ein Anion aus der Reihe $F^-$, $Cl^-$, $Br^-$, $I^-$, $CH_3O^-$, $C_2H_5O^-$, $C_3H_7O^-$, t-$C_4H_9O^-$, $C_6H_5O^-$, $HO^-$, $AcO^-$, $SCN^-$, $OCN^-$, $ClO_4^-$ trägt, in homogener Phase oder in einem Zweiphasensystem mit Wasserstoffperoxid umsetzt.

2. Verfahren nach Anspruch 1,
**dadurch gekennzeichnet,**
daß man als Reaktionsmedium ein organisches Lösungsmittel oder ein Lösungsmittelgemisch verwendet, welches einen Übertritt von als wäßrige Lösung eingesetztem Wasserstoffperoxid in die organische Phase gestattet.

3. Verfahren nach Anspruch 1 oder 2,
**dadurch gekennzeichnet,**
daß man bei Einsatz von wasserfreiem Wasserstoffperoxid als organische Lösungsmittel Alkyl- oder Cycloalkylester von gesättigten aliphatischen Carbonsäuren mit einer Kohlenstoffzahl von 4 - 8 einsetzt.

4. Verfahren nach den Ansprüchen 1 bis 3,
**dadurch gekennzeichnet,**
daß man den zur Epoxidation verwendeten, gebrauchten Katalysator nach Abtrennung der Reaktionsmischung für weitere Epoxidationen einsetzt.

## Claims

1. Process for the catalytic epoxidation of olefins with hydrogen peroxide, characterised in that the olefin is reacted with hydrogen peroxide in homogeneous phase or in a diphasic system in the presence of

– rhenium-oxo complexes or
– a dinuclear compound of the type µ-oxobis[porphyrinatooxorhenium(V)) in each case together with
– octaethylporphyrin,
– 5, 10, 15, 20-tetraphenylporphyrinor
– 5, 10, 15, 20-tetra(4-pyridyl)-porphyrin

as ligands in which hydrogen atoms or free electron pairs are optionally substituted one or several times on the phenyl or pyridyl groups by halogen, hydroxy, carboxy, cyano, thiocyano, nitro, $C_1$-$C_6$ -alkyl, trihalogenomethyl, $C_1$-$C_6$-alkoxy, $C_1$-$C_6$-alkanesulphonyloxy, aminocarbonyl, an aminocarbonyl containing one or two $C_1$-$C_6$-alkyl groups, $C_1$-$C_6$-alkylcarbonyl, amino, di-$C_1$-$C_6$-alkylamino, $C_1$-$C_6$-al kanoylamino, $C_1$-$C_6$-alkyl-$C_1$-$C_6$-alkanoylamino, $C_1$-$C_6$-alkanesulphonylamino, $C_1$-$C_6$-alkyl-$C_1$-$C_6$-al kanesulphonylamino, aminosulphonyl, an aminosulphonyl containing one or two $C_1$-$C_6$-alkyl groups, $C_1$ -$C_6$-alkoxysulponyl (-$SO_2$-O-$C_1$-$C_6$-alkyl), sulpho or $C_1$-$C_6$-alkanesulphonyl and two of these groups may be methylenedioxy groups, and in the case of the rhenium-oxo complexes the complex optionally carries on the central atom an anion from the series comprising $F^-$, $Cl^-$, $Br^-$, $I^-$, $CH_3O^-$, $C_2H_5O^-$, $C_3H_7O$, t-$C_4H_9O^-$, $C_6H_5O^-$, $HO^-$, $AcO^-$, $SCN^-$, $OCN^-$ or $ClO_4^-$.

2. Process according to Claim 1, characterised in that the reaction medium used is an organic solvent or a solvent mixture enabling the hydrogen peroxide put into the process as an aqueous solution to be transferred to the organic phase.

3. Process according to Claim 1 or 2, characterised in that when anhydrous hydrogen peroxide is used, alkyl or cycloalkyl esters of saturated aliphatic carboxylic acids having a carbon number of from 4 to 8 are used as organic solvents.

4. Process according to Claims 1 to 3, characterised in that the catalyst which has been used for epoxidation is used for subsequent epoxidations after removal of the reaction mixture.

**Revendications**

1. Procédé d'époxydation catalytique des oléfines à l'aide de peroxyde d'hydrogène caractérisé en ce que l'on met à réagir l'oléfine avec le peroxyde d'hydrogène en présence de:
– complexes oxo-rhénium ou
– de composés à deux noyaux du type μ-oxobis[porphyrinato-oxo-rhenium (V)] avec respectivement de:
– l'octaéthylporphyrine,
– la 5,10,15,20-tétraphénylporphyrine ou
– la 5,10,15,20-tétra(4-pyridyl)porphyrine
en tant que ligand, pour lesquels le cas échéant respectivement des atomes d'hydrogène ou des paires d'électrons libres sur les radicaux phényl ou pyridyle, sont substitués une ou plusieurs fois par un halogèe, un hydroxy, un carboxy, un cyano, un thiocyanato, un nitro, un alcoyle en $C_1$-$C_6$, un trihalogénométhyle, un alcoxy en $C_1C_6$, un alcane en $C_1C_6$ sulfonyloxy, un aminocarbonyl, un aminocarbonyle contenant un ou deux radicaux alcoyle en $C_1$-$C_6$, un alcoyle en $C_1$-$C_6$ carbonyle, un amino, un dialcoyle en $C_1$-$C_6$ amino, un alcanoyle en $C_1$-$C_6$ amino, un alcoyle en $C_1$-$C_6$ alcanoyle en $C_1$-$C_6$ amino, un alcane en $C_1$-$C_6$ sulfonylamino, un alcoyle en $C_1$-$C_6$ alcane en $C_1$-$C_6$ sulfonylamino, un aminosulfonyle, un aminosulfonyle contenant un ou deux radicaux alcoyle en $C_1$-$C_6$, un alcoxy en $C_1$-$C_6$ sulfonyle ($-SO_2-O-$alcoyle en $C_1$-$C_2$), un sulfo ou un alcane en $C_1$-$C_6$ sulfonyle et deux de ces radicaux peuvent être également le radical méthylènedioxy dans lesquels le complexe porte pour les complexes oxorhénium éventuellement sur l'atome central, un anion choisi dans la série constituée par $F^-$, $C1^-$, $Br^-$, $I^-$, $CH_3O^-$, $C_2H_5$, $C_3H_7O^-$, $t-C_4H_9O^-$, $C_6H_5O^-$, $HO^-$, $AcO^-$, $SCN^-$, $OCN^-$, $ClO_4^-$ en phase homogène ou dans un système à deux phases.

2. Procédé selon la revendication 1, caractérisé en ce que l'on utilise comme milieu réactionnel un solvant organique ou un mélange de solvants qui permet un passage du peroxyde d'hydrogène mis en jeu sous forme de solution aqueuse, dans la phase organique.

3. Procédé selon la revendication 1 ou 2, caractérisé en ce que l'on met en œuvre pour la mise en jeu de peroxyde d'hydrogène anhydre, en tant que solvant organique un ester alcoylique ou cycloalcoylique d'acide carboxylique aliphatique saturé, ayant un nombre d'atomes de carbone allant de 4 à 8.

4. Procédé selon les revendications 1 à 3, caractérisé en ce que l'on met en œuvre le catalyseur épuisé utilisé pour l'époxydation, pour des époxydations ultérieures, après séparation du mélange réactionnel.